# EUROPEAN PATENT SPECIFICATION

(11) **EP 2 446 341 B1**
(45) Date of publication and mention of the grant of the patent: **31.05.2017**
(21) Application number: 09818650.5
(22) Date of filing: 22.12.2009
(51) Int. Cl.: G06F 3/01

(54) **VIRTUAL REALITY INTERFACE SYSTEM**
SCHNITSTELLENSYSTEM FÜR VIRTUELLE REALITÄT
SYSTÈME D'INTERFACE DE RÉALITÉ VIRTUELLE

(30) Priority: 24.12.2008 GR 20080100821
(43) Date of publication of application: 02.05.2012
(73) Proprietor: Altoida AG, 6003 Luzern (CH)
(72) Inventor: Tarnanas, Ioannis, 8247 Flurlingen (CH); Harper, Bernard, Liverpool L18 8DQ (GB); Majer, Alan Fredrick, Toronto, ON M6J 3C2 (CA); Testerman, Randall Douglas, Clifford, NI 48727 (US)
(74) Representative: P&TS SA (AG, Ltd.)
(86) International application number: PCT/US2009/069350
(87) International publication number: WO 2010/075481

(56) References cited:
- DE-A1- 19 900 442
- US-A- 5 372 561
- US-A- 6 152 854
- GREENBERG S ET AL: "Phidgets: easy development of physical interfaces through physical widgets"[Online] 2001, pages 209-218, XP002582877 Proceedings of the 14th annual ACM Symposium on User interface software and technology - UIST '01 Retrieved from the Internet: URL:http://delivery.acm.org/10.1145/510000 /502388/p209-greenberg.pdf?key1=502388&key 2=3344135721&coll=GUIDE&dl=GUIDE&CFID=9225 2549&CFTOKEN=75133124>
- Tierney et al: "Virtual Reality in Gait Rehabilitation"[Online] September 2007 (2007-09), XP002582876 MODSIM World Retrieved from the Internet: URL:http://robot.gnu.ac.kr/paper/ieee_mech a_2010.pdf> [retrieved on 2010-05-19]
- ROGER E KAUFMAN ED - F-L KRAUSE ET AL: "A Family of New Ergonomic Harness Mechanisms for Full-Body Natural Constrained Motions in Virtual Environments" 3D USER INTERFACES, 2007. 3DUI '07. IEEE SYMPOSIUM ON, IEEE, PISCATAWAY, NJ, USA, 1 March 2007 (2007-03-01), XP031069625 ISBN: 978-1-4244-0907-5
- Thwaites: "The Immersant Experience of Osmose and Ephémère"[Online] 2005, pages 148-155, XP002582878 ICAT 2005 Retrieved from the Internet: URL:http://delivery.acm.org/10.1145/116000 0/1152427/p148-thwaites.pdf?key1=1152427&k ey2=0011724721&coll=GUIDE&dl=GUIDE&CFID=90 884189&CFTOKEN=99848324> [retrieved on 2010-05-19]
- Anonymous: "SmartNav 4:AT - Overview :: SmartNav :: Hands-Free Ergonomic Mouse :: Cursor Control :: Assistive Technology :: NaturalPoint", , 16 December 2008 (2008-12-16), XP055287060, Retrieved from the Internet: URL:https://web.archive.org/web/2008121606 1521/http://www.naturalpoint.com/smartnav/ products/4-at/ [retrieved on 2016-07-08]

## Description

### Cross Reference to Related Applications

The Present Application is based entirely upon my Greek Patent Application Serial No. 20080100821 filed on December 24, 2008, entitled "Extreme Virtual Reality Interface System." The Present Application claims the benefit of and priority to said Greek

### Patent Application.

### Background of the invention

### Technical field

The invention addresses the main challenges in the locomotion technology field by providing a low cost, novel virtual reality (VR) interface, which conveys to users a powerful sense of physical participation in a virtual world. The proposed system includes high levels of safe physical immersion, even with the user making extreme physical motions such as running, jumping, tumbling and twisting. These manoeuvres are conducted with the user immersed in a low cost virtual reality environment that has almost immediate usability for first time users without the need for extensive training in how to use it safely.

### Description of the prior art

Several virtual reality setups that simulate locomotion have already been developed and it has been shown that the sense of immersion is greatly affected by the selected approach (Slater *et al* 1998, Usoh *et al* 1999). Proposed methods range from 3D wands via "walking in place"-metaphors to treadmill systems. In general, treadmill-like devices are designed to travel in the opposite direction to the user's movement, adapting the tread's speed to keep him or her in essentially the same position. Current state-of-the-art one degree-of-freedom (DOF) treadmills, which are commonly used for fitness, rehabilitation or virtual reality, can be equipped with a tilt device and/or a tether to simulate uphill walking (e.g., the "Treadport" by Sarcos, USA, Hollerbach et al 2000), but they have the disadvantage of confining the user's movement to one direction only. At present there have only been a few attempts to enable omni-directional walking on 2D-treadmills. Two degrees-of freedom can be achieved by using two orthogonal translatory actuators ("Omni-directional treadmill" by Virtual Space Devices, USA, Mitchell 1999, and the "Torus treadmill" by H. Iwata, Japan, Iwata 1999). A similar setup is also described in Darken 1997. Another way of achieving 2D omni-directional actuation is by combining translation in one direction with rotation in the other (Noma and Miyasato, Japan, 1999). Three degrees-of freedom make it possible to control the orientation and the x/y position of the user on the simulated floor (Wang *et al,* Japan, 2003). Another solution described in Provisional patent No 2003/0198 uses a "universal wheel", in which the treads are not tyres but are composed of several freely spinning rollers which actuate the surface. Furthermore, in Roston and Peurach 1997, a design of programmable foot platforms providing 3D motion have been proposed. Similar systems are currently in prototype development at companies Cybernet Systems Corp. and Sarcos Corp. Apart from this, a different approach suggested for omni-directional walking are fully immersive spherical projection systems. Such designs are described for instance by companies VR-Systems Inc., Virtusphere Inc. or Time's Up Inc.

Although such treadmills have the advantage of being holonomic (cf. Campion *et al* 1996), these systems have not passed the early prototype stage. Furthermore, common problems with these designs are their high complexity and the noise level generated. Moreover and most importantly, current treadmill designs attempt to immediately counteract each step of the user, which generates relatively large forces due to abrupt accelerations. Consequently this leads to an unnatural walking behaviour, which is why all the attempts made to date are still unsatisfactory. In summary, there is no convincing omni-directional system available as of yet.

One very recent system called the GameRuner relies on a passive treadmill and therefore has no relevance to the system proposed by the present invention.

Another reported system is CyberCarpet from the CyberWalk project. The key idea of the CyberWalk project is to generate only gentle accelerations of the floor under the user's feet and to only pull the user in the direction towards the centre of the platform. That is, the user may take a few steps before the platform is fully accelerated (in rotation and translation) and it may take a while to stop after the user stands still. In order to achieve the goal of only producing gentle accelerations on the user, the CyberCarpet platform ideally should be very large. From a technical and financial viewpoint, however, there will be distinct limits to the size of the platform, which still have to be explored. One possibility to counteract a restricted platform size is to try using "perceptual tricks" to virtually increase the effective size of the platform. Initial work has been done in this direction (e.g. Slater et al 1993, 1995, 1998, Razzaque et al 2002, Popp et al 2004). It will be part of the CyberWalk project to further explore whether by for example introducing different gain factors for the visual display and the movement of the motion platform, one can virtually generate a larger workspace without this being noticed by the user.

US 6,152,854 A (Caremein David) relates to the field of human rehabilitation, simulation, training, exercise equipment, and generally methods that permit the user of the equipment to walk, run or crawl in any arbitrary direction and employing haptic sensing to increase a user's level of immersion in the simulated environment.

### Other prior art includes:

GREENBERG S ET AL: "Phidgets: easy development of physical interfaces through physical widgets" 2001, UIST'01 Proceedings of the 14th annual ACM symposium on User interface software and technology Pages 209-218,

Tierney et al: "Virtual Reality in Gait Rehabilitation" September 2007 (2007-09), XP002582876, and

US 5 372 561 A (LYNCH ROBERT P [US]) 13 December 1994 (1994-12-13)

Thwaites, "The Immersant Experience of Osmose and Ephémère", XP002582878, ICAT 2005

### Summary of the invention

It is an object of the present invention to provide a system which will be compact, easy to use, of low cost and which will enable high immersive virtual-locomotion by allowing: i) extreme physical movements by active breathing-controlled body weight support, ii) quick or slow movements, and iii) unrestricted omni-directional walking.

The proposed system addresses the main challenges in the locomotion technology field. It provides a low cost, novel virtual reality interface, which conveys to users a powerful sense of physical participation in a virtual world. The list of system characteristics includes:
- Augmented or superhuman capabilities - users are able to perform moves they would normally not be capable of performing.
- Reverse Interactivity - the system allows to powerfully interact with the user in the real world. For example, it could affect the elevation of the user and score impact hits.
- Multiple Immersion Cues - the display and interface offers multiple modes of interactivity to deepen the immersive experience.
- Diverse Compatibility. The interface utilizes existing equipment (such as flat screen TVs, surround sound systems), and can be programmed to recognize often unused devices such as exercise cycles, rowing machines and treadmills. This allows the users to enter VR immersion while increasing their overall fitness, but without the boredom often associated with home exercise machines.
- Natural Interface without joystick or wires, but with a depth sensing camera - this provides a compelling, intuitive, and immersive user experience.
- Freedom of movement - users are able to jump, kick/punch, and even perform back flips.
- Active physical involvement - it permits free movement where the user is actually exercising.
- Rapid response - low latency, ideally below 100 milliseconds, is essential to a compelling VR experience.
- Safety and comfort - the proposed solution is as safe as possible while minimizing unintended sources of discomfort.
- Low cost - the objective is a price point that is realistic for a gaming enthusiast to purchase for their home system.
- Compact - the footprint of the device is 2mx2m.
- Practical - the design is feasible to implement as a working prototype.
- Accommodate variety - the solution allows for a variety of users and body types.
- Body weight support and lifting - a version of body weight assisted force is addressed by the system.

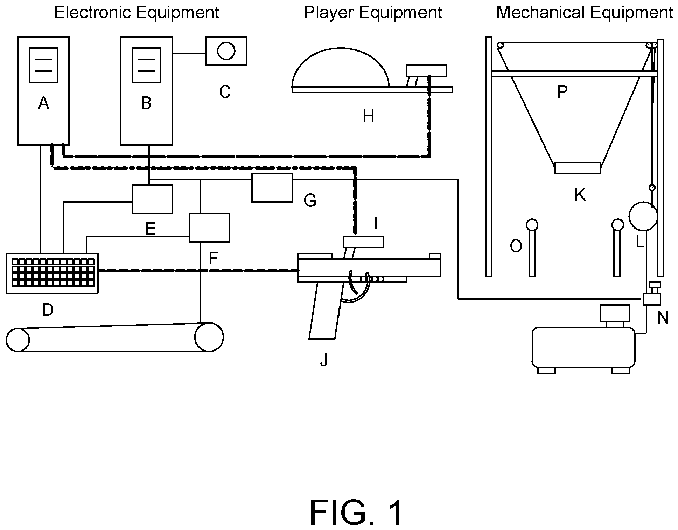

The novel features which are believed to be characteristic of the present invention together with further objects and advantages will be better understood from the following description when considered in connection with the accompanying Figure 1. It is to be expressly understood, however, that the Figure 1 is provided for the purpose of illustration and description only and is not intended as a definition of the limits of the present invention.

### Detailed description of the invention

There are three categories of equipment for the particular embodiment as depicted in Figure 1:
1) Electronic Equipment and wiring
2) Equipment worn by the user
3) Mechanical equipment and frame

The labeled parts in Figure 1 are:
A) Computer running the main software
B) Computer running depth sensing camera software, gesture recognition software, & phidget control software
C) Depth sensing camera
D) Keyboard input (soldered circuit board) for main computer
E) Phidget output to keyboard (e.g. punch and jump)
F) Phidget output to both main software and treadmill (e.g. walk and run)
G) Treadmill with electronically controlled speeds
H) "Gyro" head mouse for point-of-view control (mounted to hat), motionactivated switch
I) "Gyro" gun scope for point-of-view control
J) Optional "controller" for shooting and switching external devices (exercise bike) that also correspond to main software outputs
K) Twisting belt held by wire cables attached to air balance
L) Air balancer
M) Compressor for maintaining air balancer
N) Pressure valve/regulator for air balancer (with input for computer control)
O) Phidget output to air valve/regulator for air balance

**Not shown:** Rear projection screen and projector.

Sources of input are the depth sensing camera (C), Gyro mouse (H) and optional gun (J). The depth sensing camera connects to computer (B) which interprets the gestures and sends commands to phidgets (E & F). One phidget drives keyboard circuit (D) attached to the main computer (A) while the other connects to both keyboard circuit (D) and the treadmill (G) for run/walk commands. Meanwhile, the Gyro mouse (H) connects directly to the main computer (A) wirelessly, and is also connected to an on/off switch (I) that allows user to re-center their point of view. An optional controller (J) can also connect to the keyboard (D) allowing the user to switch and take control of another external device such as an exercise bike and perform the same task inside the virtual environment as well (e.g. switch from running to riding a bike). The final aspect is the mechanical/suspension system. This consists of a twisting belt (K) which holds the user in place and which is attached by wire cables to the air balancer (L). The latter has its lift powered by an air compressor (M). Air pressure is regulated by a valve (N). No electrical control/connections exist between the balancer/harness and the electronic aspects of the system. This feature opens up an entire range of new possibilities (allowing users to take flight using gestures for instance).

The active breathing-controlled body weight support, safety and suspension system employed by the present invention differs from the software- or computer-controlled, safety and suspension systems used in previously reported VR methods or systems. In the invention disclosed herein, a twisting belt is suspended by bungee chords that act as springs. These are to be connected to cables and pulleys arranged in a V shape. The lifting power required to raise a user beyond the highest angles is exponential. So by the present system it is almost impossible to accelerate a user upwards into a low ceiling. A further advantage is the air-balancer design employed and the additions of the "breathing" chest sensor and lifting valve. The system of the invention relies on the active user movements (breathing, balancing) and on an electronically controlled design that synchronizes the rising of the users in the real world and inside the virtual world as well. This is achieved by the chest sensor triggering both the VR elevation changes and the pressure regulation with an electronically controlled lifting valve attached to the air-balancer.

The present system incorporates the intuitive processes of breathing and balance as the primary means of navigating within the virtual world. By breathing in, the immersant is able to float upward, by breathing out, to fall, and by subtlety altering the body's centre of balance, to change direction. In conventional VR systems, the body is often reduced to a little more than a probing hand and roving eye, whereas in the system of the invention immersion depends on the body's most essential living act, that of breathing, not only to navigate, but more importantly to attain a particular state-of-being within the virtual world.

The head mouse employed by the present invention for eye gaze inside the virtual environment can be of conventional type, which is remarkably accurate device and very pleasing to use. There is only the need for it to be recalibrated when the user looks away from the screen. This can be done with a hand held trigger that is wired to the air mouse. Alternatively this function can be run wirelessly by the gun via use of an infrared triggered switch. A more sophisticated solution includes writing mouse software that is dedicated to this device and which works as follows: when the user looks away from the screen, the image is frozen at the last point the user saw and a cursor appears at the centre of the image; when the user turns his head in the general direction of the screen and is detected by the sensor, the cursor will flash for 1 second; during this second the user will look directly at the centre of the screen; when the second has elapsed, the cursor disappears and the driver software assumes that the user is looking at the middle of the screen; it then tracks the mouse motion as if it had been recalibrated using the wired switch.

Another option is to alter the controls of the wireless head mouse so that it operates in a similar fashion to a joystick. Instead of having the users' head control the absolute position of the mouse (i.e. whatever direction their head points to is where the "mouse" goes), head position could be used to control the mouse "speed" in any direction. That is, when the users' head is centred on the middle of the screen, the mouse remains still. However, the degree to which the user positions their head in any direction that is off centre, will effect the velocity of movement in that direction. For example, a slight head turn to the left will slowly move the point of view in the matching direction at a constantly low speed (as long as the users' head remains in that position), while a sharper turn left will scroll the world much faster in the same direction. Re-centring their head will then simply stop the rotation. This will easily allow the user to scroll the scene in any direction they like, and intuitively re-centre their point of view while always remaining facing forward. This will entirely eliminate the need for any external button connected to the head mouse.

In the present invention the speed of the treadmill can be controlled remotely by a depth sensing camera that is also controlling the speed of motion through a VR scene.

Another important feature is the system controller. By this a single key input controls the VR images and also a real world device such as the treadmill or an exercise bike. This provides the user with a unique level of control of the VR and the real-world experience.
The extreme virtual reality interface of the invention includes high levels of safe physical immersion, even with the user making extreme physical motions such as running, jumping tumbling and twisting. These manoeuvres are conducted with the user immersed in a low cost VR environment that has almost immediate usability for first time users without the need for extensive training in how to use it safely.

The present invention together may be better understood by referring to the accompanying example involving a specific embodiment of this invention. This example is intended for illustration purposes only and should not in any sense be construed as limiting the scope of the present invention.

### Example 1

High Immersion from a 2D, LCD/plasma Screen while playing the hypothetical game "Alien Invasion".

High levels of immersion when using small displays is difficult and requires a strong game experience coupled to an intense involvement from the user. In the home setting, the extreme VR interface (X-TVRI) works in the following way to give unprecedented levels of immersion and user interaction: a large living room contains typical home cinema elements of a 40-50 inch LCD screen and a good quality surround sound system which has been correctly calibrated. The screen is mounted mid-wall in a room that is 9 feet high, 10 feet wide and 12 feet deep (2.75m x 3.0m x 3.6m). Alternatively, a high gain cinemascope screen 1.7 metres high and 4 metres wide can be used. The screen can be hung from curved curtain rails with a centre of curvature approximately at 2.0 metres distance. The user views the VR image from the centre of curvature. This allows a vertically rolled screen to occupy the end of a typical 3 metre wide room and be folded away when not in use.

Adjacent to the screen is a home entertainment computer which is controlled by wireless keyboard. Connected to the computer is the X-TVRI which has one output connected to a small 3D depth sensing camera below the screen, the Wireless VR Controller, a compact running machine in front of the screen and the final output to the portable Suspension System. The Wireless VR Controller is used to switch external devices such as the treadmill or an exercise bike while also acting as a gun, a conventional game controller and a wand or sword. The portable Suspension System contains the chest strap to detect chest expansion which in turn has simple calibration and override controls. By sensing the chest expansion, the interface will raise the VR character and the user in the real world. The user climbs in to the twisting belt which has integrated shaping, padding and latching points, which connect to the V shape suspension system. The belt is then latched to the suspension system above the treadmill and the user is ready to begin VR immersion.

A hypothetical game is called Alien Invasion (A.I.) which is similar to Doom, Crysis and Mirrors Edge is used as an example of how X-TVRI will function as a product when launched in the public domain. "A.I." is a game where a single superhuman future soldier fights to re-take a huge spaceship from alien invaders. The soldier wears an Exo-suit that allows him to run at high speeds for long distances, punch and kick with amazing power, absorb violent attacks without serious injury, jump long distances and even fly for a few seconds when necessary. However, flying and hyper jumping uses suit power. So whenever possible, the user is encouraged to use muscle power and only use the its hyper jumping and flying as a last resort. Suit power can however be re-charged using the same power cell used for the weapons. His task is to use an on-screen navigation device to locate weapons and supplies that allow him to kill all of the Aliens before they can kill him. The aliens can locate him if he stands still or takes straight lines between way-points. So he must keep moving intelligently at all times. The user plays the role of the super soldier and begins the game without a hand-held controller. His first task is to find a virtual example of the real wireless VR controller he will need to complete the game. This is only possible because uniquely, X-TVRI has a depth sensing camera, whole body recognition software and a head mouse that allows the user to naturally navigate without the need for a hand held controller. Once he has found the virtual hand held device and enabled it, the game will only then recognise the real controller. But the virtual device needs lots of power packs that are located all over the ship. So the user will find out that if he is inefficient in his tactics or gameplay, the real gun will run out of power and he will be forced to use it as a sword, use his hands and feet as additional weapons or to run, jump and fly to escape his attackers.

The controller can be used as a sword or as a gun. When combined with the head mouse, the controller will allow the user to shoot a gun or wave a sword in an independent direction while using head and body position to navigate in a highly natural way. The head mouse accelerometer is attached to a lightweight safety helmet. The helmet is necessary because the user will be expected to be performing jumps, flips and twists that may bump his head. So the head mouse is essential in this design to ensure that the user wears the helmet to play the game. The VR begins with the soldier waking from suspended animation to the sound of an intruder alert alarm. The user looks around the VR room with small head motions and the software re-draws the scene as if much larger head motions have been used. His VR Head-Up-Display (H.U.D) switches on to tell him of the invasion and give him his instructions. He walks towards to the real TV screen and the depth sensing camera detects his motions. This starts the treadmill and moves him back to the optimum distance no matter how fast he moves. He sees a touch screen to the side of the doorway in the game. He faces the touch screen; the software detects his face direction and re-draws the VR to centre it on the real TV screen. He continues to walk in a single direction in reality. But in the VR he is now moving in a new direction. As he moves, the software is constantly redrawing the room to give him a holographic look-around effect around objects, even though he is simply viewing a large 2D screen. He stops walking and the depth sensing camera detects that in the real world he is moving away beyond the optimum distance, so it stops the treadmill. In this way, the software can allow the user to physically run and change VR direction while he actually in one location and physically running or walking in only one direction.

The user turns his head quickly a few degrees. But the scene re-draws much further and he is now facing behind himself in virtual space. The surround sound tracks the virtual world so the alien sound is directly ahead and the alarm behind him. From out of the shadows a large alien is approaching quickly. But without a weapon the user has only one option: he expands his chest and jumps a few inches upwards while lifting his feet. The suspension system senses his chest expansion, catches his leap and raises him a little. But the VR scene is re-drawn to a much greater height and the user feels as if he is surging to a high viewpoint. He leans back and kicks out at the alien's head. At the point of contact, the leg actuators are triggered and the alien is killed. The user continues to flip backwards to land on his feet. VR gravity lowers the screen viewpoint and the air balancer lowers the user to the ground after an extended period of suspension. Adrenalin pumping, the interface allows the user and the VR character to run to vertical shaft. He looks up a little while expanding his chest. The scene redraws directly upwards and the suspension system lifts him of the ground. He is now flying in any direction he faces while looking for new clues of how to continue the adventure.

His HUD tells him that a weapon and power packs can be found on the far side of the main hold. It shows him a map and plans a straight route for him to take. He runs at high speed to the ships hold and quickly arrives at a balcony overlooking the vast cargo area. His HUD tells him that the balconies that would take him around the cargo hold to his weapons are infested with aliens and suggests he run in straight line across the cargo bay. The scene below him looks like the roofs, streets and tall buildings of a life-size city.

The user looks around the scene as sees that walking along a narrow balcony rail will lead him to a point where he can drop down to the "roof tops" below without flying or hyper jumping. He looks up and the TV screen re-draws the image to show that above his head is a bar. But he can only reach it by expensive flying or if he uses a low wall to his left as a booster to gain the height needed to reach it. He takes a small step backwards and the runs forward and jumps up with his right foot landing on the right side bar. This allows him to get the extra jumping height needed to reach the real bar above his head. He hangs for while before swinging down to level of the balcony rail. He then walks along the balcony rail and follows the pathway down to the rooftops. From here the navigation is exactly like Mirrors Edge with our user running, jumping and hanging from roof to roof while actually covering a similar distance in reality running on the treadmill. However, to gain extra height in jumping the user can expand his chest to tell the suspension system to give him an extra amount of lift.

The user eventually finds the weapons store. As he solves the puzzle of how to open it, he has been tracked down by Aliens. He switches on the virtual weapon only to find a HUD notice saying the weapon will be powered up in 30 seconds. But that is too long. The first Alien attacks him and lands a blow on his face. This is achieved be making the punching hand loom massively and very quickly in the users visual field. The user's autonomic looming response will automatically make him blink his eyes and turn his head away. To simulate an actual punch the X-TVRI has a near trick: the looming response is perfectly timed to a horribly realistic punching sound while the treadmill is ran in reverse very quickly for a split second. This will cause the user to fall backwards and feel as if he has really been punched. The user gets up quickly because the suspension has been designed to catch his fall and then lift him back up onto his feet when he expands his chest. He then begins to fight using the real game controller as a sword. The VR system tracks the real sword and animates a virtual version of it on the screen. But there are too many aliens so he raises his hands above his head and expands his chest again. This tells the X-TVRI system that the user needs to fly and he is lifted off the ground in "superman mode." The system reads his body posture to then fly him in his intended direction and away from danger.

The game is now to run fast, think quickly, shoot accurately and fight fiercely when one is out of ammo or re-charging one's weapon. If the treadmill were to run at very high speeds, the user could "hyper-run" while the portable suspension system reduces foot loads to only a few kilos. Next there can be game levels that require extreme physical movements simply to progress to the next level. In this respect, the levels reality will be much higher than in conventional game. And the amount of physical energy and physical agility required to complete the course or required to fight will be an order of magnitude higher than conventional computer games. The user will also be acutely aware that the game "fights back" and that on-screen characters or events can cause exciting or unexpected effects in the real world. For instance, in this game the alien could be armed with grenades. If one lands near the feet of our user, it would be easy to simulate blowing him off his feet. The way it will work is that when a grenade explodes, the surround sound will produce an unusually loud shock wave effect from the direction of the grenade. The experience is timed so that just before the sound is due to reach his ears, the treadmill is run in reverse to knock the user backwards (like the alien punch). But this time the suspension system is timed to lift the user off his feel and keep him in the air for a time period proportional to the proximity of the explosion. In most cases, this experience is likely to kill the super soldier and lead to another type of experience in the game.

If the user is killed by the aliens, the game goes into "spirit mode." In this mode, its functions are the exact opposite of the main game. Here, the user can move around the game as a spirit and must not use any extreme body motions. The CGI visuals are all softened and the chest sensor will allow the VR character to float upwards and move through walls and floors as if they were a ghost. To move upwards, one must fill one's lungs with air to increase virtual buoyancy. To descend, one simply breathes out. To move forward or left and right, the camera will detect tiny body or head motions and amplify them as large character movements on the screen. If one moves too much, the game will freeze or maybe even shut down. In spirit mode, the user can fly around the ship and spy on the aliens before returning to the suspended animation chamber. There he can find a new super soldier body to occupy and begin the game all over again. This contrast between the highly energetic main game and the ethereal death experience is a remarkably engrossing and unique aspect to the game and X-TVRI.

### References

Bülthoff, H.H. and van Veen, H.A.H.C. (2001): Vision and action in virtual environments: modern psychophysics in spatial cognition research. In: Vision and Attention, (Eds.) J. Jenkins, L. Harris. (Springer, New York).
Campion G., Bastin G. and d'Andrea-Novel B. (1996): Structural Properties and Classification of Kinematic and Dynamic Models of Wheeled Mobile Robot, IEEE Trans. on Robotics and Automation, vol. 12, pp. 47-62.
Darken, R.P., Cockayne, W.R., & Carmein, D. (1997): The Omni-Directional Treadmill: A Locomotion Device for Virtual Worlds. Proceedings of UIST '97, pp. 213-221.
Deutscher J., Blake A. and Reid I. (2000): Articulated Body Motion Capture by Annealed Particle Filtering, CVPR, pp. 126-133.
Ernst, M. O., Banks, M. S. and Bülthoff, H. H. (2000): Touch can change visual slant perception. Nature Neuroscience 3 (1), 69-73.
Ernst, M. O. and Banks, M. S. (2002): Humans integrate visual and haptic information in a statistically optimal fashion. Nature 415, 429-433.
Hillis, J.M., Ernst, M. O., Banks, M. S. and Landy, M. S. (2002): Combining sensory information: Mandatory fusion within, but not between, senses. Science 298, 1627-1630.
Hollerbach, J.M., Xu, Y., Christensen, R., and Jacobsen, S.C. (2000): Design specifications for the second generation Sarcos Treadport locomotion interface, Haptics Symposium, Proc. ASME Dynamic Systems and Control Division, DSC-vol. 69-2, Orlando, pp. 1293-1298.
Isard M. and Blake A. (1998): CONDENSATION -- Conditional Density Propagation for Visual Tracking. International Journal on Computer Vision, vol. 1, no. 29, pp. 5-28.
Iwata H. (1999): Walking About Virtual Environments on an Infinite Floor, IEEE Virtual Reality, 13 - 17 March.
Jung, T. & Haulsen, I.: Immersive Escape-Route Scenario with Locomotion Devices, Proceedings of Workshop on Spatial Cognition in Real and Virtual Environments, Tübingen, Germany, April 1999.
Karnath H-O, Ferber S, Himmelbach M (2001): Spatial awareness is a function of the temporal not the posterior parietal lobe. Nature 411, pp. 950-953.
Lewald J, Karnath H-O (2002): The effect of whole-body tilt on sound lateralization. European Journal of Neuroscience 16, pp. 761-766.
Mitchell, A.J. (1996): "Omni-directional treadmill", International Patent Application PCT/GB97/00785, March 20, 1996.
Mittelstaedt, M.-L. & Mittelstaedt, H. (1997) The effect of Centrifugal Forces on the Perception of Rotation about the Vertical Axis. Naturwissenschaften 84, 366-369.
Niemeier M, Karnath H-O (2003): Stimulus-driven and voluntary saccades are coded in different coordinate systems. Current Biology 13, pp. 585-589.
Noma H. and Miyasato T. (1999): A New Approach for Cancelling Turning Motion in the Locomotion Interface, ATLAS", Proc of ASME-DSC-vol.67, pp.405-406.
Popp, M. M., Gouy, E., & Holtmannspötter, J. (2004): Real Walking in Virtual Environments: A new Experimental Device. EuroHaptics Conference Proceedings 2004 (Ed. Buss. M., Technische Universität München).
Provisional patent application No 2003/0198 : Dispositif de déplacement Omnidirectionnel, brevet provisionnel n° 2003/0198 depose le 26 mars 2003.
Patent Application De 10 2004016429.0 by the Max-Planck Society. Razzaque, S., Swapp, D. Slater, M. Whitton, M. C. & Steed, A. (2002). Redirected Walking in Place. Eight Eurographics Workshop on Virtual Environments (Eds, S. Müller, W. Stüzlinger)
Rehg M. and Kanade T. (1995): Model-Based Tracking of Self-Occluding Articulated Objects, CCV, pp. 612-617.
Roston, G.P., and Peurach, T. (1997). A whole body kinesthetic display device for virtual reality applications. Proc. IEEE Intl. Conf. Robotics and Automation, 3006-3011.
Schraudolph N.N. (1999): Local Gain Adaptation in Stochastic Gradient Descent, Proc. Int. Conf. Artificial Neural Networks, pp. 569-574.
Schraudolph N.N. (2002): Fast Curvature Matrix-Vector Products for Second-Order Gradient Descent, Neural Computation, vol. 14, no. 7, pp. 1723-1738.
Sidenbladh H., Black M. J. and Fleet D. J.(2000): Stochastic Tracking of 3D Human figures using 2D Image Motion. ECCV, pp. 702-718.
Slater, M., Steed, A., & Usoh, M. (1993). "The Virtual Treadmill: A Naturalistic Metaphor for Navigation in Immersive Virtual Environments," First Eurographics Workshop on Virtual Reality, ed. M. Goebel, 71-86.
Slater, M., Usoh, M., & Steed, A. (1995) "Taking Steps: The Influence of a Walking Technique on Presence in Virtual Reality," ACM Trans. on CHI, Special Issue on Virtual Reality Software and Technology, 2, 3: 201-219, September.
Slater, M., Steed, A., McCarthy, J, Maringelli, F. (1998). The Influence of Body Movement on Subjective Presence in Virtual Environment, Human Factors, 40(3), 469-477.
Sminchisescu C. and Triggs B. (2001): Covariance Scaled Sampling for Monocular 3D Body Tracking, CVPR, pp. 447-454.
Usoh, M., Arthur, K., Whitton, M.C., Bastos, R., Steed, A., Slater, M. & Brooks, Jr., F.P. (1999). Walking > Walking-in-Place > Flying, in Virtual Environments. Proceedings of SIGGRAPH 99.
Wang Z., Bauernfeind K. and Sugar T. (2003): Omni-Directional Treadmill System", VR2003, Haptics Symposium.
Wu Y., Lin J. Y. and Huang T. S. (2001): Capturing Natural Hand Articulation, ICCV, pp. 426-432.

## Claims

1. A virtual reality interface system for immersive virtual-locomotion, which comprises:
(A) a main computer running main software,
(B) a computer running gesture recognition software, depth sensing camera software and phidget control software,
(D) a keyboard comprising a soldered circuit board, for the main computer,
(G) a treadmill with electronically controlled speeds,
(C) a depth sensing camera,
(E) a phidget output to the keyboard, initiating punch, kick and/or jump actions of a character in a virtual reality environment,
(F)a phidget output to both the main software and the treadmill,
(H) a gyro, head mouse for point-of view control, mounted on a hat or lightweight safety helmet, and having a motionactivated switch,
(I) a gyro gun scope for point-of-view control, (K) a twisting belt,
(L) an air balancer, wherein the twisting belt is held by wire cables attached to the air balancer, and wherein the air balancer is configured such that the full weight of the user can be supported by the twisting belt,
- a compressor for powering the lift of the air balancer,
(N)a pressure regulator, being a valve, for the air balancer, having an input for computer control,
(O) a phidget output to the pressure regulator for the air balancer,
- a rear projection screen,
- a projector, and
- a chest sensor which is configured to detect when a user's chest has expanded and to detect when a user's chest has retracted, and wherein the chest sensor is configured to:

2. System according to claim 1, further comprising a plurality of external devices including a gun and an exercise bike, and a controller for switching between the external devices, so as that any of the external devices can be selectively used to control the performance of a corresponding activity of a character in a virtual reality environment.

## Patentansprüche

1. Ein Virtual-Reality-Interface System für immersive virtuelle Fortbewegung welches umfasst:
(A) einen Hauptrechner auf welchem die Hauptsoftware läuft,
(B) einen Rechner, auf dem Gestenerkennungssoftware, Tiefenmessungskamera-Software und Phidgetkontrollsoftware läuft,
(D) eine Tastatur, umfassend eine gelötete Leiterplatte, für den Hauptrechner,
(G) ein Laufband mit elektronisch geregelten Geschwindigkeiten,
(C) eine Tiefenmessungskamera,
(E) ein Phidget-Output zur Tastatur, der einen Faustschlag-, eine Tritt- und/oder Sprungaktionen einer Figur in einer Virtual-Reality-Umgebung auslöst,
(F) ein Phidget-Output sowohl zur Hauptsoftware als auch zum Laufband,
(H) eine Gyrohauptmaus zur Standpunkt- oder Blickrichtungskontrolle, an einem Hut oder einem leichtem Schutzhelm angebracht und mit einem bewegungsgesteuerten Schalter,
(I) ein Gyro-Waffenzielfernrohr zur Standpunkt- oder Richtungskontrolle,
(K) einen Twistinggurt,
(L) einen Luftausgleicher, wobei der Twistinggurt mittels am Air Federzug befestigten Drahtseilen gehalten wird, und
worin der Luftausgleicher so konfiguriert ist, dass das Gesamtgewicht des Benutzers vom Twistinggurt getragen werden kann,
einen Kompressor der den Hub des Luftausgleichers antreibt,
(N) einen Druckregler, der ein Ventil ist, für den Luftausgleicher mit einem Eingang für Rechnersteuerung,
(O) einen Phidget-Output zum Druckregler für den Luftausgleicher,
einen Rückprojektionsbildschirm
einen Projektor
und einen Brustsensor, der ausgebildet ist, um zu erkennen, wenn die Brust eines Benutzers sich ausgedehnt hat und zu erkennen, wenn die Brust eines Benutzers sich zusammengezogen hat und wobei der Brustsensor ausgebildet ist
den Luftausgleicher als Reaktion auf die Feststellung, dass sich die Brust des Benutzers ausgedehnt hat, zu veranlassen, den Benutzer körperlich anzuheben und gleichzeitig das Anheben einer Figur in einer virtuellen Realität zu veranlassen, und den Luftausgleicher zu veranlassen, wenn der Brustsensor erkennt, dass sich die Brust des Benutzers zusammengezogen hat, den Benutzer körperlich abzusenken und gleichzeitig das Senken der Figur in einer virtuellen Realität zu veranlassen.

2. System gemäss Anspruch 1 zudem enthaltend eine Vielzahl an externen Geräten, einschliesslich einer Pistole und eines Übungsfahrrads, und einen Kontroller um zwischen den externen Geräten umzuschalten, dergestalt dass ein beliebiges der externen Geräten selektiv benutzt werden kann, um die Leistung/Performance einer entsprechenden Aktivität einer Figur in einer in einer virtuellen Reahtät zu kontrollieren.

## Revendications

1. Un système d'interface de réalité virtuelle pour locomotion virtuelle immersive, qui comprend :
(A) un ordinateur principal exécutant le logiciel principal,
(B) un ordinateur exécutant un logiciel de reconnaissance de gestes, un logiciel de caméra de détection de profondeur et un logiciel de contrôle de phidget,
(D) un clavier comprenant un circuit imprimé soudé, pour l'ordinateur principal,
(G) un tapis roulant avec des vitesses commandées électroniquement,
(C) une caméra de détection de profondeur,
(E) une sortie de phidget vers le clavier, initiant des actions de coups de poing, coups de pied et/ou sauts d'un personnage dans un environnement de réalité virtuelle,
(F) une sortie de phidget tant pour le logiciel principal que pour le tapis roulant,
(H) une souris principale gyroscopique pour le contrôle du point de vue, montée sur un couvre-chef ou un casque de sécurité allégé, et ayant un interrupteur activé par mouvement,
(I) un viseur gyroscopique pour le contrôle du point de vue,
(K) une ceinture de torsion,
(L) un balancier aérien, dans lequel la ceinture de torsion est tenue par des câbles métalliques attachées au balancier aérien, et
dans lequel le balancier aérien est configuré pour que le poids intégral de l'utilisateur puisse être supporté par la ceinture de torsion,
un compresseur pour fournir la puissance pour lever le balancier aérien,
(N) un régulateur de pression, étant une valve, pour le balancier aérien, ayant une entrée pour contrôle d'ordinateur,
(O) une sortie de phidget pour le régulateur de pression, pour le balancier aérien,
un écran de projection arrière
un projecteur
et un capteur thoracique qui est configuré pour détecter une expansion du thorax d'un utilisateur et pour détecter une contraction du thorax d'un utilisateur, dans lequel le capteur thoracique est configuré pour activer le balancier aérien pour soulever physiquement l'utilisateur et simultanément déclencher le soulèvement d'un personnage dans la réalité virtuelle en réponse à la détection d'une expansion du thorax de l'utilisateur,
et pour activer le balancier aérien pour descendre physiquement l'utilisateur et simultanément déclencher la descente du personnage dans la réalité virtuelle lorsque le capteur thoracique détecte la contraction du thorax de l'utilisateur.

2. Système selon la revendication 1, comprenant en outre une pluralité de dispositifs externes comprenant un fusil, un vélo d'exercice, et un contrôleur pour commuter entre les dispositifs externes, en sorte que l'un quelconque des dispositifs externes peut être utilisé sélectivement pour commander l'exécution d'une activité correspondante d'un personnage dans un environnement de réalité virtuelle.
